# EUROPEAN PATENT SPECIFICATION

(11) **EP 1 161 248 B1**
(45) Date of publication and mention of the grant of the patent: **06.05.2009**
(21) Application number: 00907851.0
(22) Date of filing: 09.03.2000
(51) Int. Cl.: A61K 33/00, A61P 17/02

(54) **PHARMACEUTICAL COMPOSITION CONTAINING NITRATE SOURCE AND AN ACIDIFYING AGENT FOR TREATING OPEN WOUND AND BURNS**
PHARMAUZETISCH ZUSAMMENSETZUNG NITRATENQUELLE UND ANSÄUERUNGSMITTELN ENTHALTENDE ZUR BEHANDLUNG VON OFFENEN WUNDEN UND VERBRENNUNGEN
COMPOSITION PHARMACEUTIQUE CONTENANT UNE SOURCE DE NITRATE ET UN AGENT ACIDIFIANT POUR LE TRAITEMENT DES BLESSURES OUVERTES ET DES BRULURES

(30) Priority: 09.03.1999 GB 9905425
(43) Date of publication of application: 12.12.2001
(73) Proprietor: QUEEN MARY & WESTFIELD COLLEGE, London E1 4NS (GB)
(72) Inventor: TUCKER, Arthur Tudor, Clinical Microvascular Unit, London EC1A 7BE (GB); BENJAMIN, Nigel, St. Bartholomew's, London EC1M 6BQ (GB)
(74) Representative: Lord, Hilton David
(86) International application number: PCT/GB2000/000853
(87) International publication number: WO 2000/053193

(56) References cited:
- WO-A-95/22335
- US-A- 4 595 591
- US-A- 5 633 284
- US-A- 5 648 101
- WELLER RICHARD ET AL: "A randomized trial of acidified nitrite cream in the treatment of tinea pedis." JOURNAL OF THE AMERICAN ACADEMY OF DERMATOLOGY, vol. 38, no. 4, April 1998 (1998-04), pages 559-563, XP002111730 ISSN: 0190-9622
- ORMEROD ANTHONY DAVID ET AL: "The inflammatory and cytotoxic effects of a nitric oxide releasing cream on normal skin." JOURNAL OF INVESTIGATIVE DERMATOLOGY, vol. 113, no. 3, September 1999 (1999-09), pages 392-397, XP000925553 ISSN: 0022-202X

## Description

The present invention relates to a new pharmaceutical use of acidified nitrite contained within a delivery system which allows passage of nitric oxide to the skin as a treatment for opens, wounds and burns.

Nitric oxide [NO] is a potent vasodilator synthesised and released by vascular endothelial cells and plays an important role in regulating vascular local resistance and blood flow. In mammalian cells, NO is principally produced along with L-citrulline by the enzymatic oxidation of L-arginine. Nitric oxide is also involved in the inhibition of both platelet and leukocyte aggregation and adhesion, the inhibition of cell proliferation, the scavenging of superoxide radicals and the modulation of endothelial layer permeability. Nitric oxide also has been shown to possess anti-microbial properties, reviewed by F. C. Fang (1997) (J. Clin. Invest. 99 (12) 2818-2825 (1997)).

A potential therapeutic utility of the anti-microbial properties of NO is described in WO 95/22335. A pharmaceutical composition comprising nitrite in an inert carrier cream or ointment and salicylic acid was used to show killing of cultures containing *E. coli* and *C. albicans.* This activity was further tested against patients with fungal infection of the feet ("Athlete's Foot" or *tidea pedis*) and showed that the condition was amenable to treatment with the acidified nitrite composition. However, the composition of nitrite and organic acid caused erythema (redness) of the skin.

A transdermal patch for topical NO delivery is for instance described in US5648101.

The in-situ formed NO is useful in gastro-enterology/endoscopy, pulmonary hypertension, as nitro-vasodilators, and in male impotance.

Local treatment of superficial lesions of human skin and mucous membrane with a nitric acid or equivalent, nitrous acid and organic carboxylic acid comprising composition is discussed US459591. For the application i.a. thin porous plastic sticks are used.

In addition to internal cell-mediated production, NO is also continually released externally from the surface of the skin by a mechanism which appears to be independent of NO synthase enzyme. Nitrate excreted in sweat is reduced to nitrite by an unknown mechanism which may involve nitrite reductase enzymes which are expressed by skin commensal bacteria. Alternatively mammalian nitrite reductase enzymes may be present in the skin which could reduce nitrite rapidly to NO on the skin surface.

The production of NO from nitrite is believed to be through the following mechanism:

No₂⁻+N+⇔HNO₂ [1]

2HNO₂⇔N₂O₂+H₂O [2]

N₂O₂⇔No+No₂ [3]

Although the amount of NO generated by this physiological mechanism is not sufficient to affect skin blood flow it is clear that very large amounts of NO can be generated by the topical application of nitrite and acid.

It has now been surprisingly found that topical application to the skin of nitrite at concentrations of up to 4% in an inert carrier cream or ointment when mixed with an organic acid such as ascorbic acid (vitamin C) reacts to produce oxides of nitrogen to cause the release of nitric oxides leading to sustained vasodilation of the microcirculatory blood vessels, without significant inflammation. This new use for acidified compositions containing nitrite is a departure from the previously known uses of the composition as an anti-microbial agent. The side-effects of erythema and irritation to the skin from the acid in the composition associated with the treatment of fungal infections of the foot had been considered to suggest that the composition should not be used on broken skin or away from sites of infection needing immediate, short term therapeutic treatment. Additionally, the skin on the foot is significantly thicker and tougher than elsewhere on the mammalian body and so can endure more prolonged erythema than other thinner areas of skin elsewhere. Furthermore there is a widespread and generally accepted medical prejudice against inserting ointments or gels into open wounds or onto broken skin. Such practice is advised against because of the risk of actually causing infection or septicaemia (blood-poisoning).

The ability of the composition to cause vasodilation is also surprising because the NO molecule would not normally be expected to cross the outer layers of the skin into the inner layers of the epidermis to act on the blood vessels and micro-capillaries.

The pharmacologically acceptable acidifying agent is adapted to reduce the pH at the site of application and can include any suitable organic acid such as ascorbic acid (vitamin C), salicylic acid, acetyl salicylic acid, acetic acid or a salt or a derivative thereof in a concentration up to 20% w/w, suitably 0.25 to 10% w/w, preferably 4 to 6 % w/w. A particularly preferred concentration is 4% or 5% w/w. The preferred pH range is from pH2 to pH7, preferably pH4. Other acidifying agents include but are not limited to, ammonium or aluminium salts, phenol, benzoic acid. Inorganic acids such as hydrochloric acid may be used if sufficient dilute and/or appropriately buffered. The acidifying agent may be present as a dissolved salt or in a liquid form.

The pharmacologically acceptable source of nitrite ions may an alkaline metal nitrite or an alkaline earth metal nitrite. For example, LiNO₂, NaNO₂, KNO₂, RbNO₂, CsNO₂, FrNO₂, Be(NO₂)₂, Mg(NO₂)₂, Ca(NO₂)₂, Sr(NO₂)₂, Ba(NO₂)₂, or Ra(NO₂)₂. Alternatively, a nitrite precursor may be used as the source of the nitrite ions in the composition, such as for example a dilute solution of nitrous acid. Other sources of nitrite ions are nitrate ions derived from alkali metal or alkaline earth metal salts capable of enzymic conversion to nitrite. For example, LiNO₃, NaNO₃, KNO₃, RbNO₃, CsNO₃, FrNO₃, Be(NO₃)₂, Mg(NO₃)₂, Ca(NO₃)₂, Sr(NO₃)₂, Ba(NO₃)₂, or Ra(NO₃)₂. The concentration of the nitrate ion source may be up to 20% w/w, suitably 0.25 to 10%, preferably 4 to 6%. A particularly preferred concentration is 4% or 5% w/w.

Suitably, the final nitrite ion concentration present in the composition is up to 20% w/w, generally in the range of from 0.25% to 15% w/w, suitably 2% to 10% w/w, preferably 4 to 6 % w/w. A particularly preferred nitrite ion concentration is 4% or 5% w/w.

Ischaemic skin conditions which may benefit from the therapeutic use of a composition as defined in accordance with this aspect of the invention, include but are not limited to wounds, including skin ulcers and post-operative trauma, burns. This aspect of the invention therefore also extends to platelet and/or leukocyte aggregation and adhesion, cell proliferation, scavenging of superoxide radicals and endothelial layer permeability.

The pharmaceutical composition may be adapted for administration by any appropriate topical route, viz. transdermal. Such compositions may be prepared by any method known in the art of pharmacy, for example by admixing the active ingredient with the carrier(s) or excipient(s) under sterile conditions.

Pharmaceutical compositions adapted for transdermal administration may be presented as discrete patches intended to remain in intimate contact with the epidermis of the recipient for a prolonged period of time. For example, the active ingredient may be delivered from the patch by iontophoresis as generally described in Pharmaceutical Research, 3(6):318 (1986).

The pharmaceutical compositions may contain preserving agents, solubilising agents, stabilising agents, wetting agents, emulsifiers, sweeteners, colourants, odourants, salts (substances of the present invention may themselves be provided in the form of a pharmaceutically acceptable salt), buffers, coating agents or antioxidants. They may also contain therapeutically active agents in addition to the substance of the present invention.

Dosages of the substance of the present invention can vary between wide limits, depending upon the disease or disorder to be treated, the severity of the condition, and the age and health of the individual to be treated, etc. and a physician will ultimately determine appropriate dosages to be used.

This dosage may be repeated as often as appropriate. If side effects develop the amount and/or frequency of the dosage can be reduced or otherwise altered or modified, in accordance with normal clinical practice.

Such compositions may be formulated for human or for veterinary medicine. The present application should be interpreted as applying equally to humans as well as to animals, unless the context clearly implies otherwise.

The present invention provides a membrane comprising a pharmacologically acceptable acidifying agent and a pharmacologically acceptable source of nitrite ions or a nitrite precursor therefore. The membrane may be fully-, or partially-permeable, including semi-permeable or selectively permeable, to the passage of nitric oxide. Such membranes can prevent direct contact of the composition with the skin but can permit diffusion of nitric oxides into the skin.

This is particularly advantageous in the treatment of areas of broken skin, open wounds or serious burns. In this way the integrity of the wound area is preserved. Suitable membranes include, but are not limited to, polymeric materials such as nitrocellulose, cellulose, agarose, alginate gels, polyethylene, polyester (e.g. a hydrophilic polyester block copolymer) etc. A suitable membrane that can be used in practice is Sympatex^{™} which is composed of fibres of hydrophilic polyester block copolymer.

The invention will now be described, by way of illustration only with reference to the following examples and figures which are provided for the purposes of illustration and are not to be construed as being limiting on the invention.

FIGURE 1 shows nitric oxide diffusion through a selection of membranes where the vertical axis shows nitric oxide concentration and the horizontal axis in the time in minutes. FIGURE 1a shows the results using Saranwrap^{™} (SW-01) and FIGURE 1b shows the results using clingfilm (CF-02).

FIGURE 2 shows the diffusion effect of the treatment through a membrane on the forearm skin microcirculatory blood flow in a healthy subject. The vertical axis is blood flow, photoplethysmography (PPG) relating to microcirculatory volume and the horizontal axis is the time in minutes.

FIGURE 3 shows the diffusion effect of the treatment through a membrane on forearm skin microcirculatory blood flow in a healthy subject. The vertical axis is blood flow, laser Doppler fluximetry (LDF) relating to microcirculatory flux and the horizontal axis is the time in minutes.

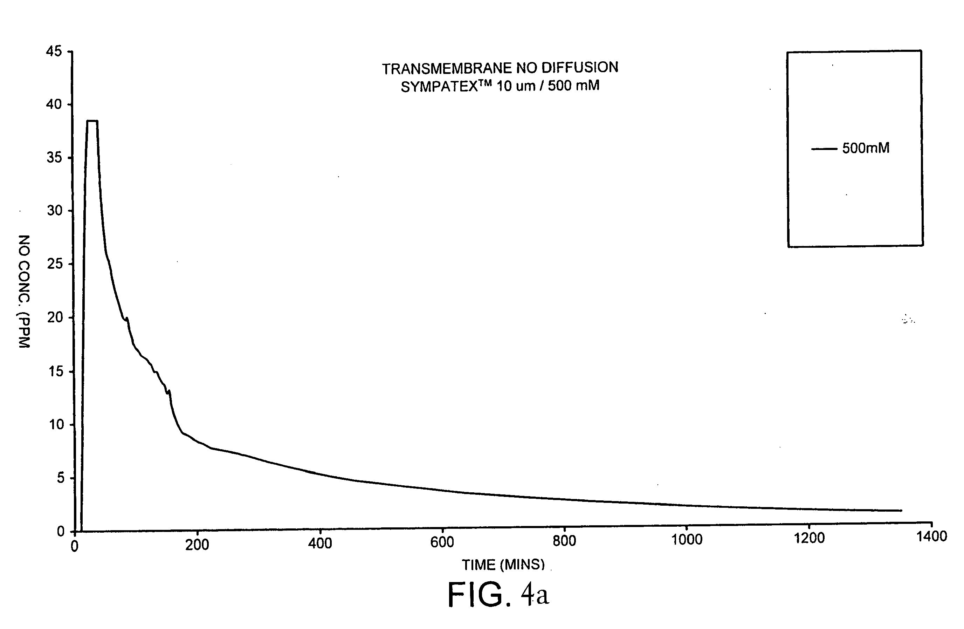

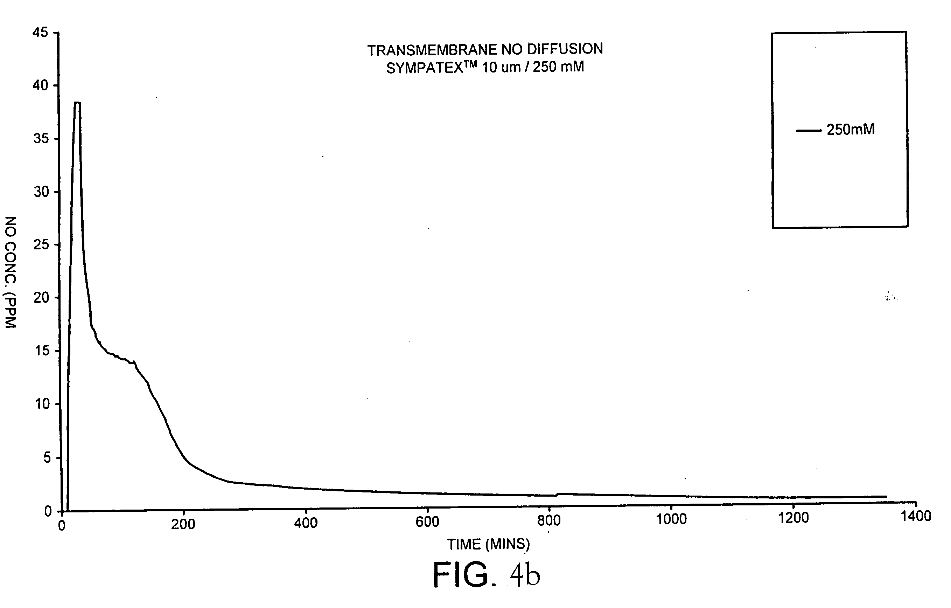

FIGURES 4 (a)-(i) show the transmembrane diffusion for sodium nitrite and ascorbic acid in 0. 8 % agar gel, using 1% sodium chloride as an intermediate at final concentrations of 500mM, 250mM, 165mM, 50mM, 25mM, 5mM, 2.5mM and 0.5mM. A control of nitrite and 0.8% agar gel using 1% sodium chloride as an intermediate was also used. The figure illustrates nitric oxide diffusion through Sympatex^{™} 10µm (Akzo Nobel) membrane where the vertical axis shows the nitric oxide concentration in parts per million (PPM) and the horizontal axis shows the time in minutes. In Figures 4(a) and 4(b) the initial peaks are artificially flattened due to the full scale deflection of the detection device.

FIGURE 5 shows the results of the application of nitric oxide generating gel consisting of 330mM of sodium nitrite and ascorbic acid in KY jelly^{™}to the forearm skin and simultaneously to SympatexTM 10µm membrane (Akzo Nobel), which was then applied to the forearm skin of the contralateral limb if nine healthy subjects. Conditions and experimental methods were the same as used for the application of the NO-generation gel on healthy subjects in Figures 2 and 3. The vertical axis shows Laser Doppler Fluximetry units and the horizontal axis shows the time in minutes.

### Example 1: Generation of nitric oxide derived through a membrane

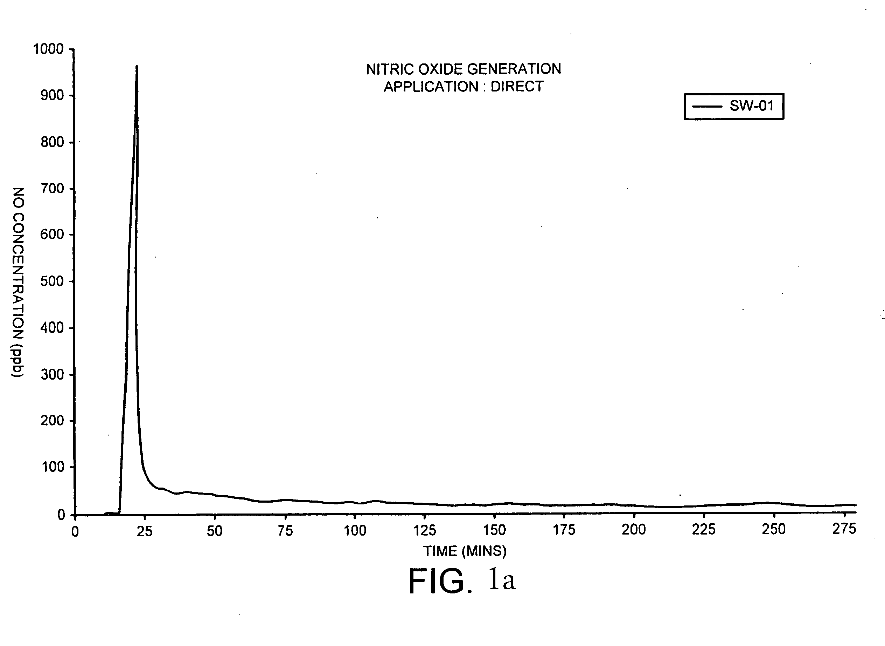

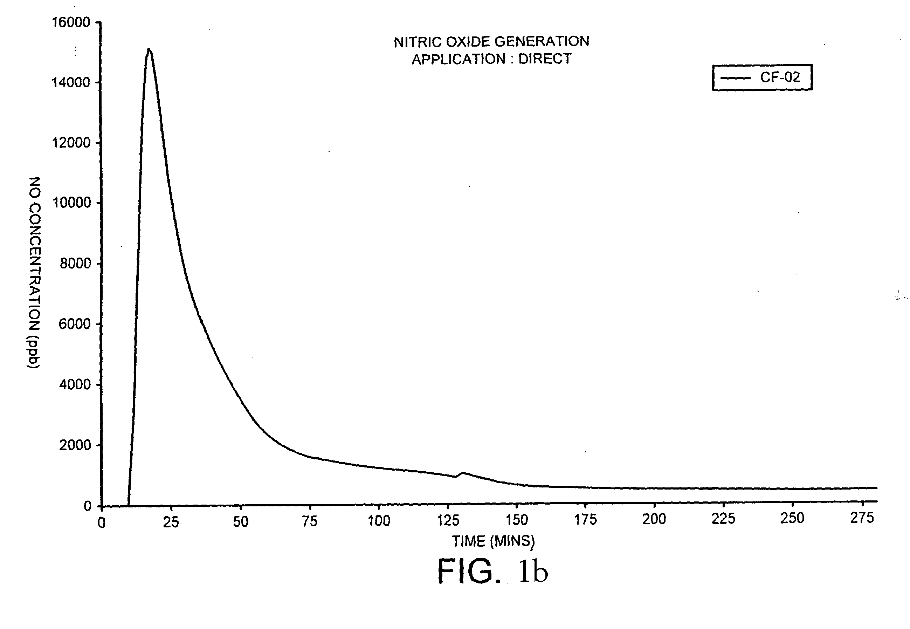

Figure 1 shows the generation of nitric oxide derived from the reaction previously detailed through a membrane. Nitric oxide concentrations were measured by a nitric oxide sensitive meter : Model 42C Chemiluminescence NO-NO₂-NOₓ analyser (Thermo Environmental Instruments Inc., MA USA) connected to a data acquisition system and IBM computer. Measurements were made continually and readings were taken every 10 seconds for 275 minutes. Material 1 was domestic clingfilm, Material 2 was Saranwrap^{™} (Sigma) and Material 3 was (Sympatex^{™}, Akzo Nobel).

### Example 2: Microcirculatory response of the application of NO-generating gel to three differing membrane materials

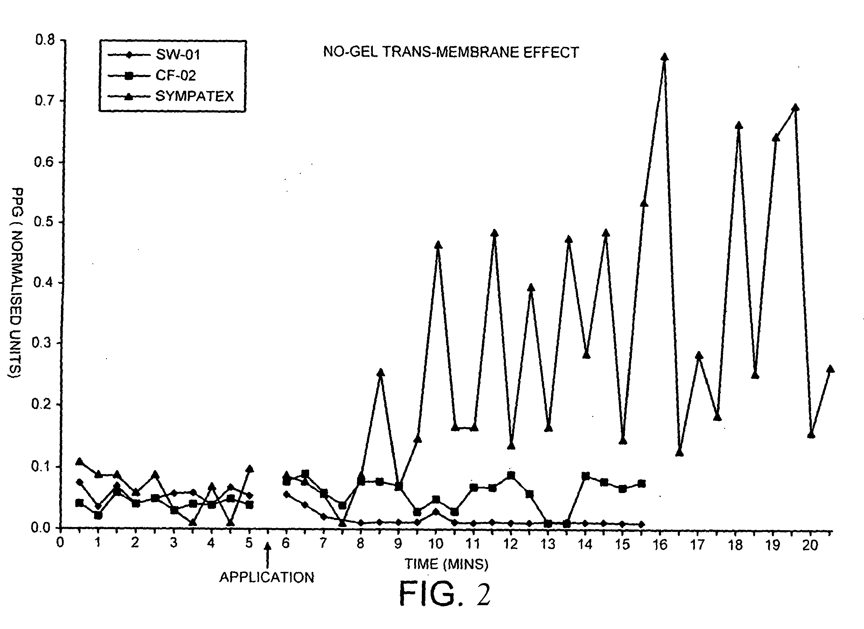

Figure 2 shows the microcirculatory response of the application of NO-generating gel to three differing membranes which were then applied to the forearm skin of a healthy subject. The skin microcirculatory volume was measured by infra-red photoplethysmography [PPG]. Material 1 was domestic clingfilm, Material 2 was Saranwrap^{™} (Sigma) and Material 3 was (Sympatex^{™}, Akzo Nobel).

The increase in microcirculatory blood volume is a reflection of the diffusion of nitric oxide through the membrane towards the skin. The transfer of nitric oxide through the membrane is a reflection of the physical characteristics of the material and is highly variable. Material number 3 (Sympatex^{™}, Akzo Nobel) had a superior diffusion profile.

### Example 3: Microcirculatory response of the application of NO-generating gel to three differing membrane materials

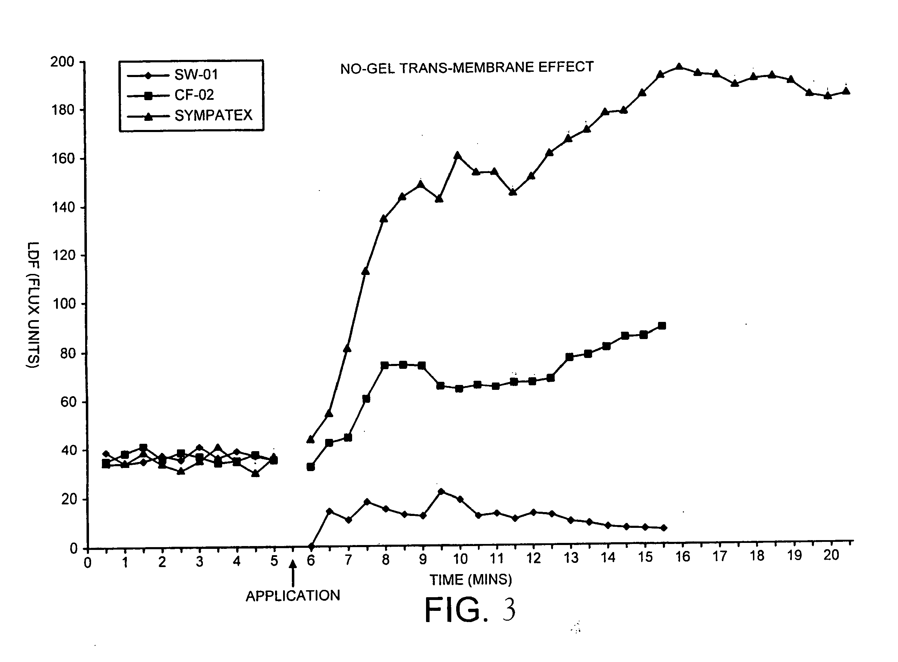

Figure 3 shows the microcirculatory response of the application of NO-generating gel to three differing membranes which were then applied to the forearm skin of a healthy subject. Conditions were the same as those used for the application of the treatment on healthy subjects in Figure 1. The skin microcirculatory velocity was measured by laser Doppler fluximetry [LDF].

The increase in microcirculatory velocity is a reflection of the diffusion of nitric oxide through the membrane towards the skin. The transfer the nitric oxide through the membrane is a reflection of the physical characteristics of the material and is highly variable. Material number 3 (Sympatex^{™}, Akzo Nobel) had a superior diffusion profile.

### Example 4: Comparison of nitric oxide generation through a membrane

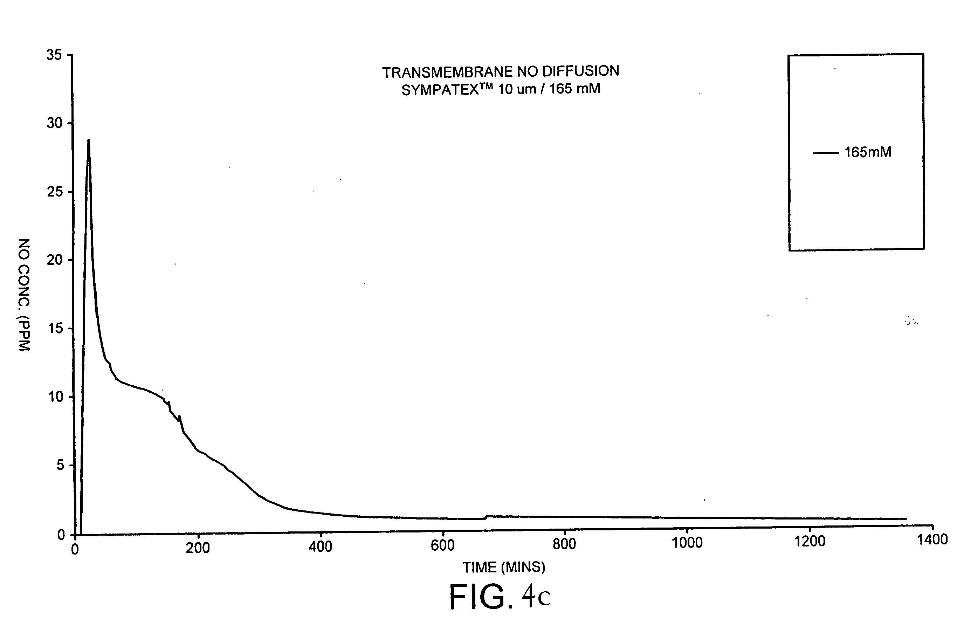

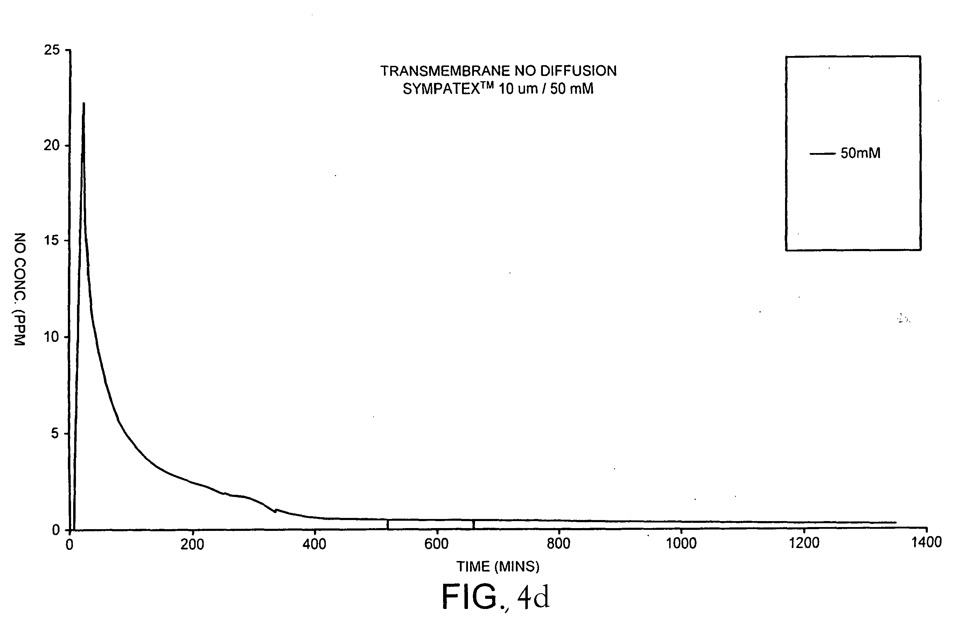

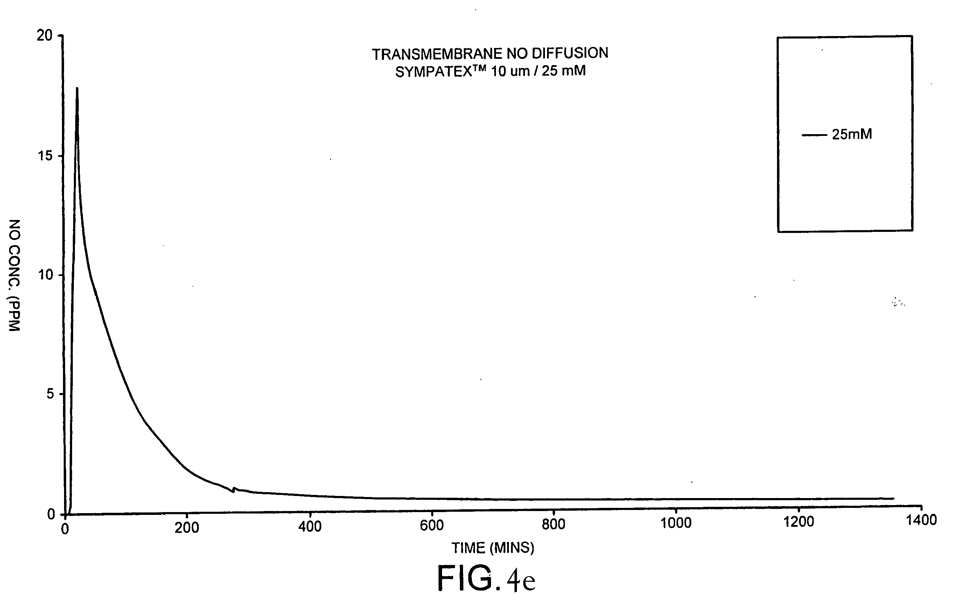

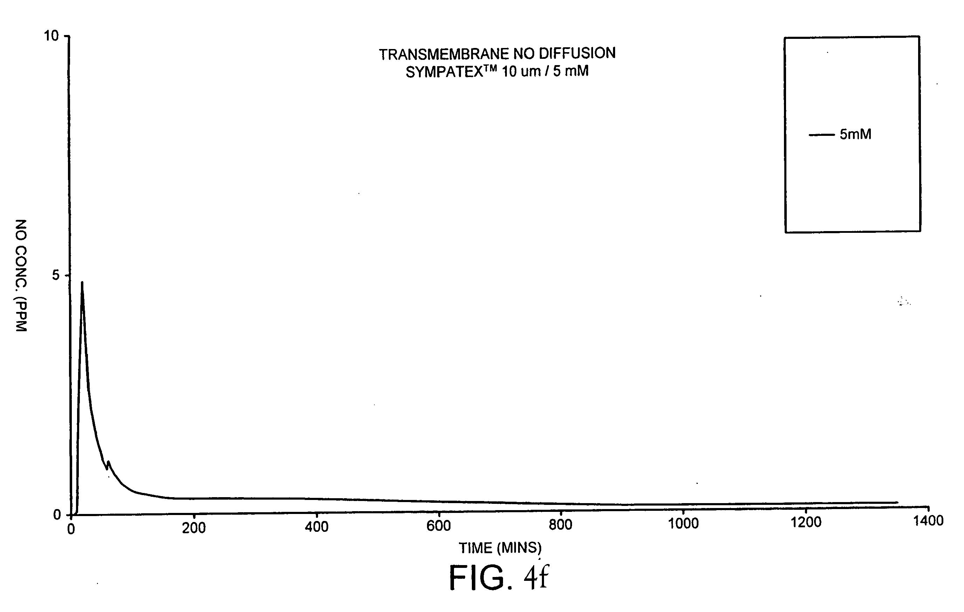

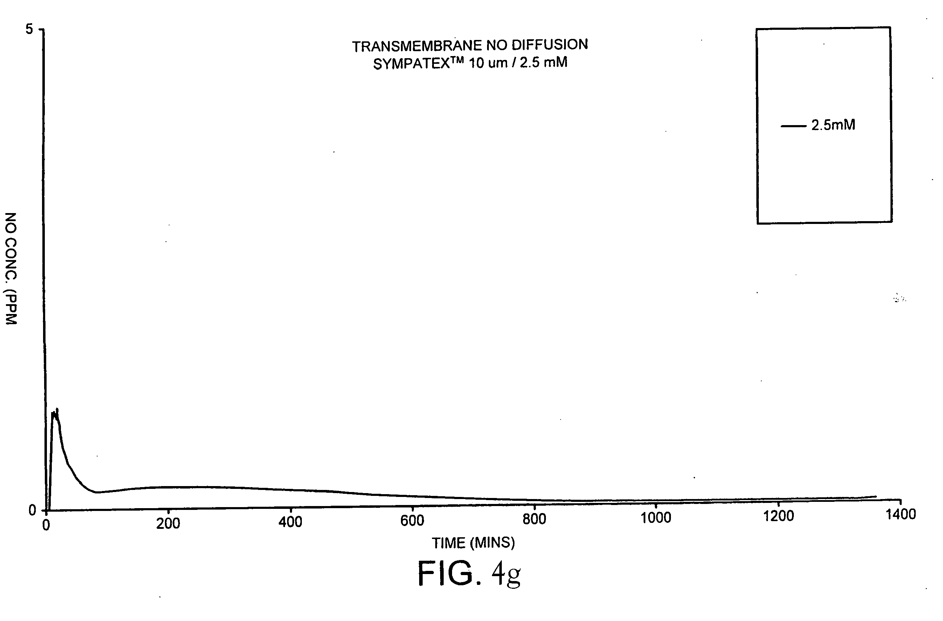

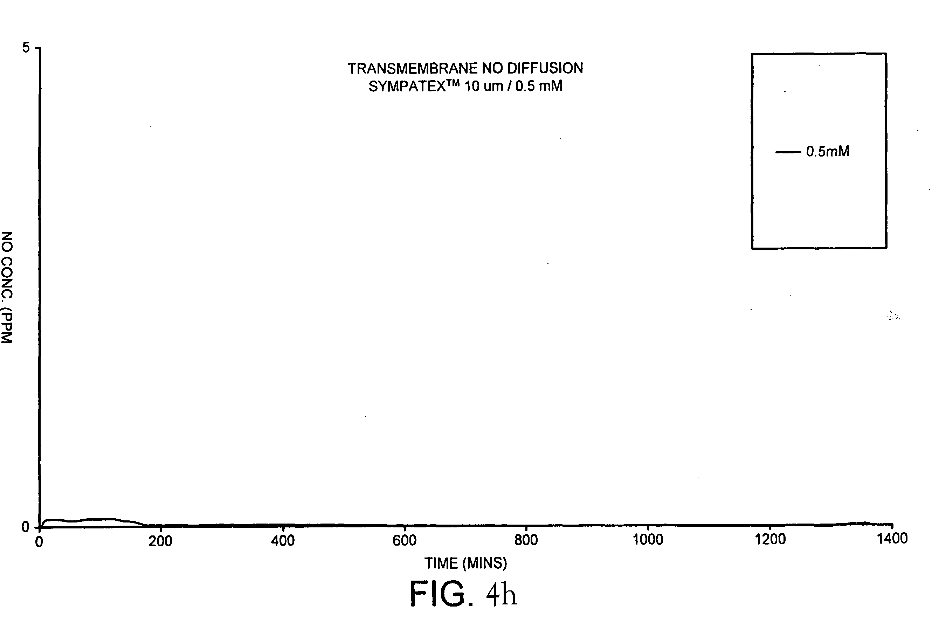

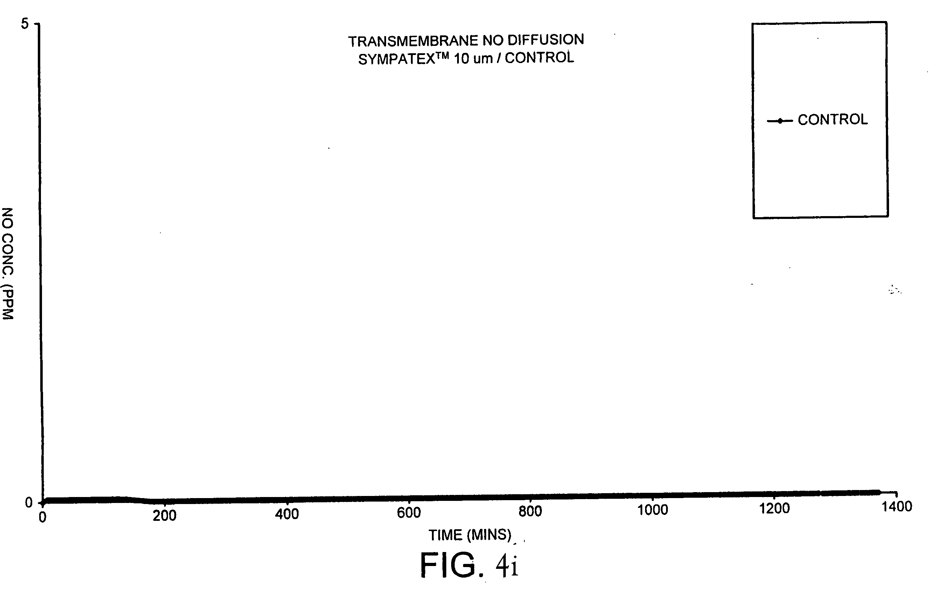

Figure 4 shows the generation of nitric oxide derived from the reaction described above through a 10µm Sympatex^{™} membrane. Nitric oxide concentrations were measured by a nitric oxide sensitive meter: Model 42C chemiluminescence NO-NO₂-NOₓ analyser (Thermo Environmental Instrumental Inc., MA, USA) connected to a data acquisition system and an IBM computer. Measurements were made continually and readings were taken every 10 seconds for 1350 minutes.

The results shown in Figure illustrate that the transmembrane diffusion coefficient is closely related to the production of nitric oxide, which is a direct product of the concentration of both the source of the nitrite ions and the acidifying agent. Furthermore, the results demonstrate that a basal production of nitric oxide is sustained for a significant period of time after mixing the reagents.

### Example 5: Microcirculatory response of the application of NO-generating gel

The nitric oxide generating gel consisting of 330mM of both sodium nitrite and ascorbic acid in KY jelly^{™} was applied directly to the forearm skin and simultaneously to SympatexTM 10µm membrane (Akzo Nobel), which was then applied to the forearm skin of the contralateral limb if nine healthy subjects. Conditions and experimental methods were the same as used for the application of the NO-generation gel on healthy subjects in Figures 1, 2, 4 and 5. The results are shown in Figure 7. It should be noted that in Figure 7 that the concentrations of the admixture are in a different unit form (i.e. mM instead of %w/w). Laser Doppler Fluximetry (LDF) measured the skin microcirculatory flux.

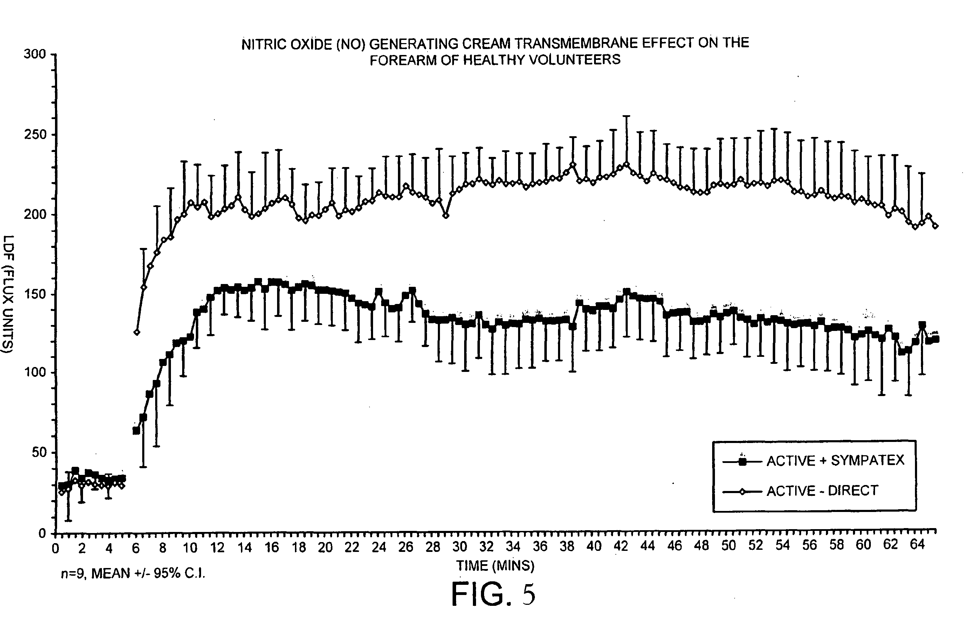

The statistically significant increase in microcirculatory flux from baseline was a reflection of the diffusion of nitric oxide through the membrane towards the skin. This vasodilation, indicated by LDF through the membrane ranged from 60-75 % (mean 64 %) of that observed when the NO-generation gel was applied directly to the skin of the forearm. The results shown in Figure 5 support the observations that the vasodilator response to the direct treatment reached a plateau phase in all patients within 10 minutes of gel application. A plateau phase, although reduced in amplitude was achieved within 16 minutes when the NO-generation gel was applied to the membrane and reflects a lag phase which is related to membrane diffusion characteristics.

## Claims

1. The use of a composition comprising an acidifying agent and an alkaline metal nitrite or an alkaline earth metal nitrite in the manufacture of a medicament for the treatment of open wounds and bums, wherein the acidifying agent is an organic acid or a salt or a derivative thereof and is adapted to reduce the pH at the site of application to from pH 2 to pH 7 and wherein the medicament further comprises a membrane, wherein said membrane is fully or partially permeable to the passage of nitric oxide and prevents direct contact of the composition with the skin.

2. The use according to claim 1, wherein the acid is selected from ascorbic acid, salicylic acid, acetyl salicylic acid, and acetic acid.

3. The use according to Claim 1 or 2, wherein the open wound is a skin ulcer.

4. The use according to any preceding claim, wherein the acid is present in a concentration up to 20% w/w.

5. The use according to Claim 4, wherein the acid is present in a concentration of 0.25% w/w to 10% w/w.

6. The use according to Claim 5, wherein the acid is present in a concentration of 4% w/w to 6 % w/w.

7. The use according to any of claims 1-3, wherein the acid is adapted to reduce the pH at the site of application to between pH2 and pH4.

8. The use according to Claim 7, wherein the acid is adapted to reduce the pH at the site of application to pH4.

9. The use according to any preceding claim, wherein the nitrite is sodium nitrite.

10. The use according to any preceding claim, wherein the nitrite is present in a concentration of up to 20% w/w.

11. The use according to Claim 10, wherein the nitrite is present in a concentration of 0.25% w/w to 15% w/w.

12. The use according to Claim 11, wherein the nitrite is present in a concentration of 2% w/w to 10% w/w.

13. The use according to Claim 12, wherein the nitrite is present in a concentration of 4% w/w to 6% w/w.

14. The use according to any preceding claim, wherein the membrane is a polymeric material.

15. The use according to any preceding claim, wherein the membrane is selected from nitrocellulose, cellulose, agarose, alginate gels, polyethylene, and polyester.

## Patentansprüche

1. Verwendung einer Zusammensetzung umfassend ein Ansäuerungsmittel und ein alkalisches Metallnitrit oder ein Erdalkalimetallnitrit bei der Herstellung eines Medikaments für die Behandlung von offenen Wunden und Verbrennungen, wobei das Ansäuerungsmittel eine organische Säure oder ein Salz oder ein Derivat derselben und geeignet ist, den pH-Wert an der Anwendungsstelle von pH 2 auf pH 7 zu reduzieren und wobei das Medikament des Weiteren eine Membran umfasst, wobei die Membran vollständig oder teilweise für den Durchgang von Stickstoffmonoxid durchlässig ist und den direkten Kontakt der Zusammensetzung mit der Haut verhindert.

2. Verwendung nach Anspruch 1, wobei die Säure unter Ascorbinsäure, Salicylsäure, Acetylsalicylsäure und Essigsäure ausgewählt wird.

3. Verwendung nach Anspruch 1 oder 2, wobei die offene Wunde ein Hautgeschwür ist.

4. Verwendung nach einem der vorhergehenden Ansprüche, wobei die Säure in einer Konzentration von bis zu 20 Gew.-% vorliegt.

5. Verwendung nach Anspruch 4, wobei die Säure in einer Konzentration von 0,25 Gew.-% bis 10 Gew.-% vorliegt.

6. Verwendung nach Anspruch 5, wobei die Säure in einer Konzentration von 4 Gew.-% bis 6 Gew.-% vorliegt.

7. Verwendung nach einem der Ansprüche 1-3, wobei die Säure geeignet ist, den pH-Wert an der Anwendungsstelle auf pH 2 bis pH 4 zu reduzieren.

8. Verwendung nach Anspruch 7, wobei die Säure geeignet ist, den pH-Wert an der Anwendungsstelle auf pH 4 zu reduzieren.

9. Verwendung nach einem der vorhergehenden Ansprüche, wobei das Nitrit Natriumnitrit ist.

10. Verwendung nach einem der vorhergehenden Ansprüche, wobei das Nitrit in einer Konzentration von bis zu 20 Gew.-% vorliegt.

11. Verwendung nach Anspruch 10, wobei das Nitrit in einer Konzentration von 0,25 Gew.% bis 15 Gew.-% vorliegt.

12. Verwendung nach Anspruch 11, wobei das Nitrit in einer Konzentration von 2 Gew.-% bis 10 Gew.-% vorliegt.

13. Verwendung nach Anspruch 12, wobei das Nitrit in einer Konzentration von 4 Gew.-% bis 6 Gew.-% vorliegt.

14. Verwendung nach einem der vorhergehenden Ansprüche, wobei die Membran ein polymeres Material ist.

15. Verwendung nach einem der vorhergehenden Ansprüche, wobei die Membran unter Nitrocellulose, Cellulose, Agarose, Alginatgelen, Polyethylen und Polyester ausgewählt wird.

## Revendications

1. Utilisation d'une composition comprenant un agent acidifiant et un nitrite de métal alcalin ou un nitrite de métal alcalino-terreux dans la fabrication d'un médicament pour le traitement de plaies ouvertes et de brulures, l'agent acidifiant étant un acide organique ou un sel ou un dérivé de celui-ci et étant adapté pour réduire le PH au site d'application de pH2 à pH 7, le médicament comprenant en outre une membrane, ladite membrane étant entièrement ou partiellement perméable au passage de l'oxyde nitrique et prévenant un contact direct de la composition avec la peau.

2. Utilisation selon la revendication 1, dans laquelle l'acide est sélectionné à partir de l'acide ascorbique, l'acide salicylique, l'acide acétyl-salicylique, et l'acide acétique.

3. Utilisation selon la revendication 1 ou 2, dans laquelle la plaie ouverte est un ulcère de la peau.

4. Utilisation selon l'une quelconque des revendications précédentes, dans laquelle l'acide est présent dans une concentration de jusqu'à 20 % en poids.

5. Utilisation selon la revendication 4, dans laquelle l'acide est présent dans une concentration de 0,25 % en poids à 10 % en poids.

6. Utilisation selon la revendication 5, dans laquelle l'acide est présent dans une concentration de 4 % en poids à 6 % en poids.

7. Utilisation selon l'une quelconque des revendications 1 à 3, dans laquelle l'acide est adapté pour réduire le pH au site d'application à entre pH2 et pH4.

8. Utilisation selon la revendication 7, dans laquelle l'acide est adapté pour réduire le pH au site d'application à ph4.

9. Utilisation selon l'une quelconque des revendications précédentes, dans laquelle le nitrite est du nitrite de sodium.

10. Utilisation selon l'une quelconque des revendications précédentes, dans laquelle le nitrite est présent dans une concentration de jusqu'à 20 % en poids.

11. Utilisation selon la revendication 10, dans laquelle le nitrite est présent dans une concentration de 0,25 % en poids à 15% en poids.

12. Utilisation selon la revendication 11, dans laquelle le nitrite est présent dans une concentration de 2 % en poids à 10 % en poids.

13. Utilisation selon la revendication 12, dans laquelle le nitrite est présent dans une concentration de 4 % en poids à 6 % en poids.

14. Utilisation selon l'une quelconque des revendications précédentes, dans laquelle la membrane est un matériau polymérique.

15. Utilisation selon l'une quelconque des revendications précédentes, dans laquelle la membrane est sélectionnée parmi la nitrocellulose, la cellulose, l'agarose, les gels d'alginate, le polyéthylène et le polyester.
